# EUROPÄISCHE PATENTSCHRIFT

(11) **EP 3 713 543 B1**
(45) Veröffentlichungstag und Bekanntmachung des Hinweises auf die Patenterteilung: **04.11.2026**
(21) Anmeldenummer: 18814500.7
(22) Anmeldetag: 21.11.2018
(51) Int. Cl.: A61K 9/00

(54) **TASCHENFÖRMIGE ORAL AUFLÖSENDE FILME MIT HOHER WIRKSTOFFBELADUNG**
POUCH-TYPE ORALLY DISSOLVING FILMS WITH HIGH ACTIVE INGREDIENT CONCENTRATION
FILMS À DISSOLUTION ORALE EN FORME DE POCHE À FORTE CHARGE EN PRINCIPE ACTIF

(30) Priorität: 21.11.2017 DE 102017127434
(43) Veröffentlichungstag der Anmeldung: 30.09.2020
(73) Patentinhaber: LTS Lohmann Therapie-Systeme AG, 56626 Andernach (DE)
(72) Erfinder: SCHMITZ, Christoph, 56598 Rheinbrohl (DE); BAUER, Marius, 56626 Andernach (DE)
(74) Vertreter: Meissner Bolte Partnerschaft mbB
(86) Internationale Anmeldenummer: PCT/EP2018/082094
(87) Internationale Veröffentlichungsnummer: WO 2019/101800

(56) Entgegenhaltungen:
- WO-A1-2016/090075
- WO-A2-02/02085
- WO-A2-2007/138484

## Beschreibung

Die Erfindung betrifft Verabreichungsformen für Wirkstoffe mit einem Hohlraum, in dem der Wirkstoff vorliegt, wobei die Verabreichungsform wasserlöslich ist, so dass sie sich beim Einnehmen im Mund rasch auflöst und den Wirkstoff freisetzt. Dadurch, dass der Wirkstoff in einem Hohlraum der Verabreichungsform eingebracht werden kann, werden technische Beschränkungen für das Einbeziehen größerer Wirkstoffmengen in typische Dünnfilm-Formulierungen umgangen, so dass unproblematisch auch größere Wirkstoffmengen in die Verabreichungsform einbezogen werden können. Die vorliegende Erfindung betrifft weiterhin Verfahren zur Herstellung entsprechender Verabreichungsformen.

Zur Verabreichung von Wirkstoffen über die Mundschleimhaut werden üblicherweise Buccal- oder Sublingualtabletten verwendet, die den Wirkstoff im Mundraum freisetzen. Die Resorption des Wirkstoffs über die Mundschleimhaut bietet gegenüber anderen peroralen Darreichungsformen einige Vorteile, beispielsweise, dass der Wirkungseintritt aufgrund einer Umgehung der Magen-Darm-Passage rasch erfolgt und dass die Wirkstoffausnutzung hoch ist.

Ein weiteres Problem von Tabletten oder Kapseln besteht darin, dass diese in der Regel geschluckt werden, was bedingt, dass der Patient eine Flüssigkeit bereithält, mit der er diese Darreichungsform zu sich nehmen kann. Teilweise bestehen jedoch bei älteren Patienten oder Kindern Schluckbeschwerden, so dass diese die Einnahme von Tabletten oder Kapseln ablehnen bzw. eine Einnahme nur ungern erfolgt. Zudem ist es möglich, dass Tabletten und Kapseln vom Patienten längere Zeit im Mund behalten und dann ausgespuckt werden. Hieraus resultiert dann nicht selten eine schlechte Compliance, die sich nachteilig beim Heilungsfortschritt bzw. Therapieerfolg bemerkbar macht.

Als alternative Darreichungsform zu den bekannten Buccal- und Sublingualtabletten sind flächenförmige-oblatenartige Darreichungsformen bekannt, die auch als Wafer bezeichnet werden. So beschreibt beispielsweise die US 5,529,782 ein schnell lösliches Filmprodukt aus löslichem Polymermaterial oder komplexen Polysacchariden, das vornehmlich zur Verabreichung von Kontrazeptiva eingesetzt wird. Das Filmprodukt soll eine Dicke von 3 bis 4 mm aufweisen und seine Löslichkeit soll so einstellbar sein, dass es sich innerhalb von 5 bis 60 Sekunden nach Verabreichung aufgelöst hat. Das Filmprodukt kann auch in Form eines Laminats vorliegen, welches mit Gas aufgeschäumte Hohlräume aufweist.

Aus der EP 0 450 141 B1 ist ein Trägermaterial zur Verabreichung von Arzneimitteln bekannt, welches sich bei Kontakt mit Speichel schnell auflöst. Bei diesem Trägermaterial handelt es sich um einen porösen, dehydratisierten, skelettartigen Trägerstoff, insbesondere auf Basis von Proteinen und Polysacchariden. Die durch Dehydratisierung erzeugten Hohlräume werden für das Einbringen von flüssigen Wirkstoffen genutzt.

In der WO 00/18365 wird eine essbare Folie vorgeschlagen, die schnell löslich sein soll, aber auch gut an der Mundschleimhaut haften kann, um antimikrobielle Substanzen abzugeben und die Zahl unerwünschter Mikroorganismen in der Mundflora verringert. Bei den antimikrobiellen Substanzen handelt es sich zum Beispiel um ätherische Öle, die als lipophile Phase vorzugsweise mit Pullulan als Matrixmaterial in der wässrigen Phase vermischt werden.

Die WO 02/02085 beschreibt schnell zerfallende Darreichungsformen zur Freisetzung von Wirkstoffen im Mundraum oder anderen Körperöffnungen, wobei die Darreichungsform eine Matrix aufweist, die zumindest ein wasserlösliches Polymer als Grundsubstanz enthält und die mit Hohlräumen versehen ist.

Auch orale, sich im Mundraum schnell auflösende Filme (OTF (= oral thin film)-Systeme) müssen so formuliert werden, dass der Film bestimmten physikalischen Anforderungen genügt. So müssen derartige Filme beispielsweise eine gewisse Mindestfestigkeit aufweisen, damit sie bei der Handhabung durch den Patienten nicht brechen. Ein weiteres Problem bei OTF-Formulierungen besteht darin, dass die Filme nicht in beliebiger Dicke hergestellt werden können, da die wesentliche Eigenschaft der Filme darin besteht, dass sie sich im Mund rasch auflösen. Dies ist jedoch bei relativ dicken Filmen nicht mehr gewährleistet, da der Zutritt von Wasser oder Speichel in den inneren Bereich des Films bei einer höheren Dicke erschwert ist.

Darüber hinaus sind OTF-Systeme nicht nur in Bezug auf ihre Dicke, sondern auch in Bezug auf ihre maximale Größe beschränkt, da der Anwender den Film ohne Probleme in den Mund und auf die Zunge legen können soll; dies wäre bei sehr großen Filmen nicht mehr möglich. Aufgrund dieser Rahmenbedingungen ist die zu applizierende Wirkstoffmenge bei normalen filmförmigen OTF-Formulierungen auf etwa 20 mg beschränkt.

Dies stellt zum einen bei Wirkstoffen ein Problem dar, die in höheren Mengen appliziert werden müssen, zum anderen ist dies jedoch auch ein Problem bei bitteren oder anderen als geschmacklich unangenehm empfundenen Wirkstoffen, da diese in der Regel mit signifikanten Mengen an geschmacksmaskierenden Mitteln formuliert werden müssen. Auch in diesem Fall ist aber die Gesamtmenge des Wirkstoffs und gegebenenfalls zusätzlicher geschmacksmaskierender Mittel in einer OTF-Formulierung auf etwa 20 mg beschränkt.

Vor diesem Hintergrund besteht ein Bedarf für eine Verabreichungsform von Wirkstoffen, die die gleichen Vorteile wie die bekannten OTF-Formulierungen aufweist, d.h. insbesondere ein rasches Sichauflösen und Freisetzen des Wirkstoffs im Mundraum, andererseits aber keiner so starken Beschränkung in Bezug auf die mögliche zu applizierende Wirkstoffmenge unterliegt.

Ein weiteres Problem bei den bekannten OTF-Systemen besteht darin, dass zur Herstellung der Filme Wirkstoffe mit dem eingesetzten Matrixmaterial vermischt werden müssen, wozu entweder ein Lösungsmittel eingesetzt werden kann oder eine Vermischung im Rahmen eines Extrusionsverfahrens erfolgt. Bei einer Verarbeitung mit Hilfe von Lösungsmitteln muss dieses Lösungsmittel im weiteren Verlauf des Verfahrens aus dem System entfernt werden, wozu das System in der Regel erhitzt wird. Dies stellt bei Temperatur labilen Wirkstoffen ein Problem dar, da sich während der Verdampfung des Lösungsmittels die in das OTF integrierten Wirkstoffe zersetzen können. Alternativ kann bei den Lösungen das Lösungsmittel auch unter einem leichten Vakuum entfernt werden. Dies erfordert jedoch geeignete Apparaturen und ist aus technischer Sicht nur mit einem höheren Aufwand zu realisieren, was Kostennachteile mit sich bringt.

Bei einem Extrusionsverfahren werden die Wirkstoffe ebenfalls einer höheren Temperatur ausgesetzt, was zu einer teilweisen Zersetzung des Wirkstoffs führen kann.

Vor diesem Hintergrund besteht auch ein Bedarf für eine Verabreichungsform für einen Wirkstoff, die hergestellt werden kann, ohne dass der Wirkstoff hohen Temperaturen ausgesetzt werden muss. Dadurch sollen Verabreichungsformen hergestellt werden können, die auch mit temperaturlabilen Wirkstoffen beladen werden können.

Die WO 2007/138484 A2 beschreibt lösliche Tabakfilmstreifen zum oralen Verzehr, die eine Tabakkomponente, ggf. ein Geschmacksmittel, ein Bindemittel und ein Feuchthaltemittel enthalten. Der Tabakfilmstreifen kann in einer Ausführungsform aus zwei Lagen eines wasserlöslichen Films gebildet sein, zwischen denen Tabakpartikel eingebracht sind, oder als Tasche, in die geschnittener Tabak eingebracht wird.

Die WO 2016/090075 offenbart ein als Tasche ausgeformtes Produkt als textilem Material, die das ebenfalls Tabak als Genussmittel eingebracht ist. Das Textile Material ist gegenüber löslichen Komponenten des Tabaks permeabel, so dass diese Komponenten aus der Tasche freigesetzt werden wenn diese in den Mund gelegt wird.

Die vorliegende Erfindung befasst sich mit diesem Bedarf.

Zur Lösung der vorstehend geschilderten Aufgabe schlägt die vorliegende Erfindung gemäß Anspruch 1 eine Verabreichungsform für einen Wirkstoff zur Auflösung in der Mundhöhle vor, der eine erste Filmschicht und eine über der ersten Filmschicht angeordnete zweite Filmschicht umfasst, wobei die Zusammensetzung der ersten Filmschicht mit der der zweiten Schicht identisch sein kann und ein wasserlösliches Polymer umfasst, wobei die erste und die zweite Filmschicht über ihre überlappenden Kanten unter Bildung mindestens eines Hohlraums miteinander verbunden sind und wobei der Hohlraum mit einem pharmazeutischen Wirkstoff gefüllt ist. In den beanspruchten Verabreichungsformen ist Nikotin als pharmazeutischer Wirkstoff ausgeschlossen.

Die erfindungsgemäße Verabreichungsform besteht demzufolge im Wesentlichen aus einer durch zwei übereinander angeordneten Filmschichten gebildeten Tasche oder Beutel, die/der durch die Verbindung der Filmschichten im Kantenbereich gebildet wird. In den Hohlraum der Tasche oder des Beutels kann dann ein pharmazeutischer Wirkstoff eingebracht werden. Da die beiden Filmschichten analog regulären OTF-Formulierungen wasserlösliche Polymere umfassen, weisen sie im Vergleich zu regulären OTF-Formulierungen ähnliche Auflösungseigenschaften auf. Gegenüber diesen bieten die erfindungsgemäßen Verabreichungsformen jedoch den Vorteil, dass der pharmazeutische Wirkstoff erst nach der Trocknung der Filme eingebracht werden kann, so dass eine direkte thermische Belastung des pharmazeutischen Wirkstoffs, beispielsweise infolge der Trocknung der Filme, vermieden wird.

Die Begriffe "Tasche" und "Beutel" haben im Kontext der folgenden Beschreibung synonyme Bedeutung.

Die Angabe "zwei übereinander angeordneten Filmschichten" beinhaltet sowohl Ausführungsformen, bei denen zwei separate Filmschichten übereinander angeordnet werden, als auch Ausführungsformen, die erzeugt werden indem zwei Filmschichten durch Knicken eines Films übereinander positioniert werden.

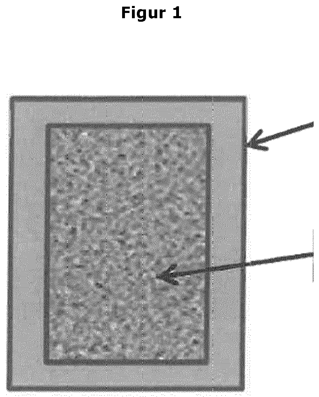

Ein Beispiel einer erfindungsgemäßen Verabreichungsform ist in Figur 1 gezeigt, in der 1 den Kantenbereich bezeichnet, über den die beiden Filmschichten miteinander verbunden sind, während 2 den mit pharmazeutischem Wirkstoff gefüllten Hohlraum zeigt.

Dadurch, dass die Verabreichungsform als "wasserlösliche" Tasche ausgebildet wird, lassen sich in dem Hohlraum auch wesentlich größere Mengen an pharmazeutischem Wirkstoff und/oder zusätzlichen Hilfsstoffe einbringen. Ein abschließendes Verschließen der Tasche nach dem Einbringen des pharmazeutischen Wirkstoffs kann über nur eine Kante der Verabreichungsform erfolgen, wofür eine Versiegelung nur im Randbereich der Verabreichungsform durchgeführt werden muss, so dass der in der Mitte der Verabreichungsform befindliche pharmazeutische Wirkstoff keiner direkten thermischen Belastung ausgesetzt werden muss.

Unter einem "wasserlöslichen Polymer" sind im Kontext der vorliegenden Erfindung wasserlösliche und/oder wasserquellbare Polymere zu verstehen, die sich in feuchter und wässriger Umgebung, wie dem Mundraum, rasch auflösen und zerfallen und so einen in die Verabreichungsform einbezogenen pharmazeutischen Wirkstoff freisetzen.

Die Angabe, dass die erste und die zweite Filmschicht "über ihre überlappenden Kanten unter Bildung mindestens eines Hohlraums miteinander verbunden sind", ist so zu verstehen, dass sich die erste und die zweite Filmschicht im Bereich ihrer Fläche (sofern der Hohlraum nicht gefüllt ist) zwar berühren können, in diesem Bereich aber nicht miteinander verbunden sind, so dass die beiden Filmschichten in diesem Bereich durch Einführen eines Materials (insbesondere des pharmazeutischen Wirkstoffes) ohne Aufwand von einander separiert werden können. Die Angabe schließt auch runde Ausführungsformen der Filmschichten ein, wobei in diesem Fall nur eine überlappende Kante vorhanden ist, die aber, um ein Einfüllen eines pharmazeutischen Wirkstoffs zu ermöglichen, nicht über ihren gesamten Umfang verbunden ist.

Für den "Hohlraum" gilt, dass dieser den pharmazeutischen Wirkstoff enthält aber im Wesentlichen frei von wasserlöslichem Polymer ist. Zudem enthält der Hohlraum bevorzugt keine kontinuierliche Formulierung des Wirkstoffs, die vollflächig mit der ersten und zweiten Filmschicht in Kontakt steht, sondern enthält den pharmazeutischen Wirkstoff in einer Form in der diskrete Gasräume zwischen einzelnen Wirkstoffpartikel vorhanden sind.

Die Verbindung der ersten Filmschicht mit der zweiten Filmschicht kann zweckmäßig durch Verkleben oder Versiegeln erfolgen. Beim Verkleben kann zum Beispiel ein geeigneter Klebstoff in den Zwischenraum zwischen der ersten und zweiten Filmschicht eingebracht werden und die erste Filmschicht so auf der zweiten Filmschicht befestigt werden. Für ein Versiegeln können die erste Filmschicht und die zweite Filmschicht erhitzt und gegeneinander gepresst werden, so dass im Bereich der Versiegelung die erste Filmschicht auf der zweiten Filmschicht haftet.

Als geeigneter wasserlöslicher Klebstoff für die Verbindung der Klebeschichten kann z.B. Plastoid E35H (weichgestelltes Eudragit E100; als Modifikatoren werden Laurinsäure, Adipinsäure und Glycerin zugesetzt) angegeben werden.

Ein weiterer geeigneter wasserlöslicher Klebstoff ist ein Klebstoff auf Basis von mindestens einem wasserlöslichen Polymer und mindestens einem Weichmacher, wobei es sich bei dem wasserlöslichen Polymer bevorzugt um Schellack, ein Vinylpyrrolidon-/Vinylacetat-Copolymer, ein Polyvinylcaprolactam/Polyvinylacetat-/Polyethylenglykol-Copolymer, Hydroxypropylcellulose oder Hydroxypropylmethylcellulose und/oder Polyvinylpyrrolidon handelt. Als geeignete Weichmacher für die Kombination mit dem wasserlöslichen Polymer können Glycerin, Polyethylenglykol, insbesondere Polyethylenglykol 200, Sorbitol und/oder Tributylcitrat angegeben werden. Bevorzugt ist der Weichmacher ausgewählt aus Glycerin, Polyethylenglykol 200 und/oder Tributylcitrat.

In Bezug auf das Verhältnis von wasserlöslichem Polymer zu Weichmacher unterliegt der Klebstoff keinen relevanten Beschränkungen, solange das Verhältnis so eingestellt ist, dass die Mischung ausreichend klebrig und verarbeitbar ist. Als günstiges Mischungsverhältnis kann ein Verhältnis von wasserlöslichem Polymer zu Weichmacher von etwa 85 bis 50 zu etwa 15 bis 50, bevorzugt 85 bis 65 zu etwa 15 zu 35, noch bevorzugter etwa 80 bis 60 zu etwa 20 bis 40, noch weiter bevorzugt etwa 80 bis 50 zu etwa 20 bis 50, noch weiter bevorzugt etwa 82 bis 68 zu etwa 18 zu 32 und am meisten bevorzugt etwa 80 bis 70 zu etwa 20 bis 30 angegeben werden.

Vorstehend wurde bereits erwähnt, dass die Zusammensetzung der ersten Filmschicht mit der der zweiten Filmschicht identisch sein kann. Da dieses zu einer Vereinfachung der Herstellung der erfindungsgemäßen Verabreichungsform führt, ist es im Rahmen der vorliegenden Erfindung bevorzugt, wenn die Zusammensetzung der ersten Filmschicht und der zweiten Filmschicht identisch ist.

Andererseits kann es in bestimmten Fällen sinnvoll sein, wenn die erste Filmschicht und die zweite Filmschicht auf unterschiedlichen Zusammensetzungen beruhen. So kann es zum Beispiel wünschenswert sein, eine der Filmschichten als mucoadhäsive Schicht auszubilden, während die zweite Schicht in wässriger Umgebung relativ schnell löslich ist, so dass der pharmazeutische Wirkstoff freigesetzt wird. In einer anderen Ausführungsform kann es zweckmäßig sein, wenn die erste Filmschicht als mucoadhäsive Filmschicht ausgebildet ist und sich die zweite Filmschicht in der Mundhöhle langsamer auflöst als die erste Filmschicht.

Hinsichtlich der Form des pharmazeutischen Wirkstoffs unterliegt die vorliegende Erfindung keinen wesentlichen Beschränkungen. So kann der pharmazeutische Wirkstoff in flüssiger oder fester Form vorliegen, wobei als feste Formen pulverförmige, granulatförmige, mikro- oder nanopartikuläre oder mikro- oder nanoverkapselte Formen besonders zweckmäßig sind. Liegt der pharmazeutische Wirkstoff in flüssiger Form vor, so liegt er jedoch möglichst nicht in wässriger Lösung oder Suspension vor, da dies die Integrität der umgebenden Filmschichten beeinträchtigt. Liegt der pharmazeutische Wirkstoff in flüssiger Form vor, sollte diese Form die angrenzenden Filmschichten möglichst nicht angreifen. Pharmazeutische Wirkstoffe in fester Form sind bevorzugt, wobei für feste Formulierungen solche auf Basis von lipophilen Basismaterialien, in denen der pharmazeutische Wirkstoff gelöst oder dispergiert ist, möglichst auszuschließen sind. Demzufolge liegt der pharmazeutische Wirkstoff in einer bevorzugten Ausführungsform nicht als liphophile nicht als ölige oder wachsartige Formulierung vor.

Unter "mikropartikulär" wird im Kontext der vorliegenden Erfindung ein Material verstanden, bei dem 90 Gew.-% und bevorzugt 95 Gew.-% der Partikel eine Partikelgröße im Bereich von weniger als 1 mm bis 1 µm aufweisen. Unter "nanopartikulär" wird im Kontext der vorliegenden Erfindung ein Material verstanden, bei dem 90 Gew.-% und bevorzugt 95 Gew.-% der Partikel eine Partikelgröße im Bereich von weniger als 1 µm aufweisen.

Bei den Begriffen "mikro- oder nanoverkapselt" bezieht sich die vorstehende Angabe jeweils auf die verkapselten Partikel.

Wie vorstehend angedeutet, besteht der wesentliche Vorteil der erfindungsgemäßen Verabreichungsform darin, dass diese auch das Einbeziehen relativ großer wirkstoffhaltiger Füllungen erlaubt. So ist es beispielsweise bevorzugt, wenn die Verabreichungsform eine Menge an wirkstoffhaltiger Füllung aufweist, die größer ist als etwa 20 mg und insbesondere größer als etwa 30 mg.

Als zweckmäßige Obergrenze für den Gehalt an wirkstoffhaltiger Füllung kann eine Menge von 1000 mg angegeben werden. Bevorzugt ist eine Obergrenze von 500 mg, weiter bevorzugt 200 mg und noch weiter bevorzugt 100 mg.

Bei einer Ausführung mit tiefgezogenen Folien, oder großen Pockets kann die Menge an wirkstoffhaltiger Füllung auch noch größer sein. Die Mengen sind dann neben der Größe des Pockets auch von der Tiefe der durch Tiefziehen hergestellten Kavität abhängig.

Als besonders günstiger Bereich für die wirkstoffhaltige Füllung kann eine Menge von etwa 50 bis etwa 200 mg angegeben werden.

Die Größe der erfindungsgemäßen Verabreichungsform ist zweckmäßig zur Aufnahme einer entsprechenden Menge an Wirkstoff-haltiger Füllung dimensioniert. Als grobe Vorgabe kann hier eine Fläche im Bereich von etwa 1 bis etwa 10 cm², und bevorzugt etwa 1,5 bis etwa 6 cm² angegeben werden. Liegt die Verabreichungsform z.B. als rechteckige Tasche vor, so kann diese Dimensionen von etwa 2 x 2,5 cm oder etwa 1 x 1,5 cm aufweisen.

Die erfindungsgemäße Verabreichungsform ist in der Regel von dünner und flacher oder leicht gewölbter Gestalt, beispielsweise in Form von kleinen Taschen, Beuteln, Tütchen, Briefchen oder Pads. Diese kleinen Taschen, Beutel, Tütchen, Briefchen oder Pads können verschiedenartige geometrische Formen darstellen, zum Beispiel kreisförmig, ellipsenförmig, länglich oder eckig, wie insbesondere rechteckig oder quadratisch.

Die Dicke der Filmschichten beträgt vorzugsweise etwa 0,01 bis etwa 2 mm, besonders bevorzugt liegt sie im Bereich von etwa 0,02 bis etwa 0,5 mm.

In Bezug auf das wasserlösliche Polymer unterliegt die vorliegende Erfindung keinen relevanten Beschränkungen mit der Maßgabe, dass das wasserlösliche Polymer ein pharmazeutisch akzeptables Material sein sollte. Geeignete wasserlösliche Polymere sind beispielsweise Stärke und Stärkederivate, Dextrane; Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose; Polyacrylsäure, Polyacrylate, Polyvinylpyrrolidone, Polyvinylalkohol, Polyethylenoxidpoylmere, Polyacrylamide, Polyethylenglycol, Gelatine, Collagen, Alginate, Pektine, Pullulan, Traganth, Chitosan, Alginsäure, Arabinogalaktan, Galaktomannan, Agar, Agarose, Carrageen und natürliche Gumme. Besonders bevorzugt im Kontext der vorliegenden Erfindung sind wasserlösliche Polymere, die aus der Gruppe, umfassend Polyvinylalkohol, Polyethylenglycol, Polyethylenoxid, Cellulosederivate, Pullulan, Gelatine und Agar, ausgewählt sind. Meist bevorzugt ist im Kontext der vorliegenden Erfindung ist Polyvinylalkohol als wasserlösliches Polymer.

Der Anteil an wasserlöslichem Polymer in der ersten und zweiten Filmschicht beträgt in der Regel etwa 85 bis etwa 100 Gewichts-%, insbesondere etwa 90 bis etwa 99,9 Gewichts-% und meist bevorzugt etwa 95 bis etwa 99,5 Gewichts-%.

Da der Polymerfilm im Gegensatz zu klassischen OTF-Systemen keinen Wirkstoff enthalten muss, kann der Anteil an wasserlöslichem Polymer sehr hoch liegen. Andererseits können, abhängig vom beabsichtigtem Anwendungsergebnis, Zusatzstoffe wie geschmacksmaskierende Mittel oder ein Teil des pharmazeutischen Wirkstoffs in die erste Filmschicht und/oder in die zweite Filmschicht einbezogen werden. In diesem Fall kann der Anteil an wasserlöslichem Polymer in der ersten Filmschicht und in der zweiten Filmschicht geringer sein als vorstehend angegeben, er sollte sich aber dennoch im Bereich von etwa 15 bis etwa 75 Gew.-% und bevorzugt etwa 50 bis etwa 70 Gew.-% bewegen.

Bei dem pharmazeutischen Wirkstoff kann es sich, mit Ausnahme von Nikotin, grundsätzlich um jeden oral verabreichbaren pharmazeutischen Wirkstoff handeln. Pharmazeutische Wirkstoffe, die im Kontext der vorliegenden Erfindung für orale Applikationen geeignet sind, sind beispielsweise antiallergische Mittel, anti-arrhythmische Mittel, Antibiotika, antidiabetische Mittel, anti-epileptische Mittel, Antihistaminika, Antitussiva, cardiotronische Mittel, Diuretika, blutdrucksenkende Mittel, Betäubungsmittel, Nervenmuskelblocker und Sexualhormone, wie Vasopressoren. Spezifische Beispiele sind Acetaminophen, Adrenalin, Alprazolam, Amlodipin, Anastrozol, Apomorphin, Aripiprazol, Atorvastatin, Baclofen, Benzocain, Benzocain/Menthol, Benzydamin, Buprenorphin, Buprenorphin/Naloxon, Buprenorphin/Naloxon/Cetirizin, Cetirizin, Chlorpheniramin, Clomipramin, Dexamethason, Dextromethorphan, Dextromethorphan/Phenylephrin, Diclofenac, Diphenhydramin, Diphenhydramin/Phenylephrin, Donepezil Dronabinol, Epinephrin, Escitalopram, Famotidin, Fentanyl, Glimepirid, GLP-1 Peptiden, Granisetron, Insulin, Insulin Nanopartikel, Insulin/GLP-1 Nanopartikel, Ketoprofen, Ketotifen, Koffein, Levocetirizin, Loperamid, Loratadin, Meclizin, Methylphenidat, Midazolam, Mirodenafil, Montelukast, Multimeric-001, Naloxon, Nitroglycerin, Olanzapin, Olopatadin, Ondansetron, Oxybutynin, Pektin, Pektin/Menthol, Pectin/Ascorbinsäure, PediaSUNAT (Artesunat und Amodiaquin), Piroxicam, Phenylephrin, Prednisolon, Pseudoephedrin, Risperidon, Rivastigmin, Rizatriptan, Selegiline, Senna Glykosiden, Sildenafil Zitrat, Simethicon, Sumatriptan, Tadalafil, Testosteron, Triamcinolon Azetonid, Triptan, Tropicamide, Voglibose, Zolmitriptan, Zolpidem, oder pharmazeutisch akzeptable Salze dieser Verbindungen. Zusätzlich kann die erfindungsgemäße Verabreichungsform nicht pharmazeutische Wirkstoffe z.B. Wirkstoffe für die Mundhygiene, wie Menthol, enthalten. Der pharmazeutische Wirkstoff kann auch eine Mischung von unterschiedlichen Wirkstoffen sein.

Die erfindungsgemäße Verabreichungsform kann neben dem bereits erwähnten wasserlöslichen Polymer als Bestandteil der ersten und zweiten Filmschicht sowie einem pharmazeutischen Wirkstoff, der sich im Hohlraum zwischen der ersten und zweiten Filmschicht befindet, weitere Inhaltsstoffe enthalten, insbesondere Hilfsstoffe, die ausgewählt sind aus der Gruppe, umfassend Farbstoffe, Aromastoffe, insbesondere Geschmacks- und/oder Geruchsstoffe, Süßstoffe, geschmacksmaskierende Mittel, Tenside, Enhancer, pH-Regulatoren, Konservierungsmittel und/oder Antioxidationsmittel. Die genannten Hilfsstoffe können Bestandteil einer oder beider Filmschichten sein und/oder mit dem pharmazeutischen Wirkstoff zusammen in den Hohlraum zwischen den beiden Filmschichten eingebracht werden.

Besonders vorteilhaft ist der Zusatz von Geschmacks-, Geruchs- und Aromastoffen, einzeln oder in Kombination.

Ein geschmacksmaskierendes Mittel ist ein Ionenaustauscherharz.

Ionentauscherharze, die zur Verwendung in der erfindungsgemäßen Darreichungsform bevorzugt sind, sind wasserunlöslich und bestehen aus einer pharmakologisch inerten organischen oder anorganischen Matrix, die kovalent gebundene funktionelle Gruppen enthält, die ionisch sind oder unter den geeigneten Bedingungen des pH-Werts ionisiert werden können. Die organische Matrix kann synthetisch sein (z. B. Polymere oder Copolymere von Acrylsäure, Methacrylsäure, sulfoniertes Styrol, sulfoniertes Divinylbenzol) oder teilweise synthetisch (z. B. modifizierte Cellulose und Dextrane). Die Matrix kann auch anorganisch sein, z.B. Kieselgel, modifiziert durch Zugabe von ionischen Gruppen. Die kovalent gebundenen Ionengruppen können stark sauer sein (z.B. Sulfonsäure), schwach sauer (z.B. Carbonsäure), stark basisch (z.B. quartäres Ammonium), schwach basisch (z.B. primäres Amin) oder eine Kombination von sauren und basischen Gruppen. Im Allgemeinen sind jene Arten von Ionenaustauschern, die zur Verwendung bei der Ionenaustauschchromatographie und für Anwendungen, wie die Deionisierung von Wasser, geeignet sind, zur Verwendung in den erfindungsgemäßen Darreichungsformen geeignet.

Bei dem Ionentauscherharz handelt es sich bevorzugt um ein Harz auf Basis von vernetztem Polystyrol. Das Polystyrol wird mit einem Vernetzungsmittel vernetzt, das aus difunktionellen Verbindungen ausgewählt ist, die in der Lage sind, Polystyrole zu vernetzen. Vorzugsweise ist das Vernetzungsmittel eine Divinyloder Polyvinylverbindung. Am meisten bevorzugt ist das Vernetzungsmittel Divinylbenzol.

Generell ist das Polystyrol zweckmäßig in einem Ausmaß von etwa 3 bis etwa 20%, vorzugsweise etwa 4 bis etwa 16%, bevorzugter etwa 6 bis etwa 10% und am meisten bevorzugt etwa 8 Gew.-%, bezogen auf das gesamte Polystyrol, vernetzt. Das Polystyrol wird mit dem Vernetzungsmittel durch bekannte Mittel vernetzt.

Im Rahmen der vorliegenden Erfindung als geschmacksmaskierende Mittel besonders geeignete Ionentauscherharze haben Austauschkapazitäten unter etwa 6 Milliäquivalenten pro Gramm (meq/g) und vorzugsweise unter etwa 5,5 meq/g.

Die Größe der Ionenaustauscherharzpartikel sollte vorzugsweise in den Bereich von etwa 20 bis etwa 200 Mikrometer fallen. Partikelgrößen deutlich unterhalb der unteren Grenze sind in allen Schritten der Verarbeitung schwer zu handhaben. Teilchengrößen oberhalb der Obergrenze, z.B. handelsübliche Ionenaustauscherharze mit sphärischer Form und Durchmessern bis zu etwa 1000 Mikrometern sind in flüssigen Dosierungsformen kiesig und haben eine stärkere Tendenz zum Bruch, wenn sie Trockenhydratisierungszyklen ausgesetzt werden.

Repräsentative Harze, die in dieser Erfindung verwendbar sind, umfassen AMBERLITE IRP-69 (erhältlich von Dow Chemical) und Dow XYS-40010. 00 (erhältlich von Dow Chemical). Beide sind sulfonierte Polymere aus Polystyrol, vernetzt mit 8% Divinylbenzol, mit einer Ionenaustauschkapazität von etwa 4.5 bis 5.5 meq/g Trockenharz (H ± Form). Ihr wesentlicher Unterschied liegt in ihrer physischen Form. AMBERLITE IRP-69 umfasst unregelmäßig geformte Partikel mit einem Größenbereich von 47 bis 149 Mikrometer, hergestellt durch Fräsen der übergeordneten, großflächigen Kugeln von AMBERLITE IRP-120. Das Dow XYS-40010. 00-Produkt umfasst kugelförmige Partikel mit einem Größenbereich von 45 bis 150 Mikrometern. Ein weiteres nützliches Austauschharz, Dow XYS-40013.00, ist ein Polymer, das aus Polystyrol besteht, das mit 8% Divinylbenzol vernetzt ist und mit einer quaternären Ammoniumgruppe funktionalisiert ist. Seine Austauschkapazität liegt normalerweise im Bereich von etwa 3 bis 4 meq/g trockenem Harz. Ein weiteres geeignetes Harz ist AMBERLITE IRP-64.

Ein geschmacksmaskierendes Mittel kann sowohl als Bestandteil der ersten und/oder der zweiten Filmschicht vorliegen, es kann jedoch auch, wie vorstehend ersichtlich, in den Hohlraum der erfindungsgemäßen Verabreichungsform eingebracht werden. Handelt es sich bei dem geschmacksmaskierenden Mittel um ein Ionentauscherharz, so ist zu beachten, dass dieses nur dann wirksam ist, wenn der pharmazeutische Wirkstoff im Beisein des Ionenaustauscherharzes in Lösung gebracht wird. Daher kann z.B. mit Formulierungen, in denen ein Ionentauscherharz als Bestandteil der ersten oder zweiten Filmschicht formuliert ist, während der pharmazeutische Wirkstoff in den Hohlraum der erfindungsgemäßen Verabreichungsform eingebracht wird, kein geschmacksmaskierender Effekt erzielt werden. Ionentauscherharze als geschmacksmaskierende Mittel sollten daher im selben Bestandteil der erfindungsgemäßen Verabreichungsform formuliert sein, wie der pharmazeutische Wirkstoff, wobei der pharmazeutische Wirkstoff zweckmäßig über eine ionische Bindung an das Ionentauscherharz gebunden ist.

Wenn die erfindungsgemäße Verabreichungsform ein geschmacksmaskierendes Mittel enthält, kann dieses in eine oder die Filmschichten einbezogen werden, oder im Fall eines mehrschichtigen Filmaufbaus in eine oder die äußeren Schichten der Filme. Dadurch lässt sich zum Beispiel eine im Vergleich zur Freisetzung des pharmazeutischen Wirkstoffs vorzeitige Freisetzung des geschmacksmaskierenden Mittels aus einer äußeren Polymerschicht realisieren, so dass die Geschmacksrezeptoren, zum Beispiel gegen bitter schmeckende pharmazeutische Wirkstoffe im Inneren der erfindungsgemäßen Verabreichungsform, bereits abgesättigt werden können, bevor der pharmazeutische Wirkstoff freigesetzt wird.

Darüber hinaus kann die erste und/oder die zweite Filmschicht mindestens ein Pigment oder ein UV-absorbierendes Mittel enthalten, das einen lichtempfindlichen in den Hohlraum der Verabreichungsform eingebrachten pharmazeutischen Wirkstoff vor UV-Licht schützt. Zudem ist es zweckmäßig, wenn die erste und/oder die zweite Filmschicht einen oder mehrere Farbstoffe, Geschmacksstoffe oder Süßungsmittel enthält.
Neben den bereits im Vorstehenden erwähnten Hilfsstoffen kann die erste Filmschicht und/oder die zweite Filmschicht auch weitere Bestandteile zur Optimierung ihrer Flexibilitäts- oder anderer physikalischer Eigenschaften enthalten, wie beispielsweise mindestens einen Weichmacher und/oder ein Feuchthaltemittel. Bevorzugte Weichmacher und/oder Feuchthaltemittel sind im Kontext der vorliegenden Erfindung z.B. ausgewählt aus der Gruppe, umfassend Glycerin, Propylenglycol, Polyethylenglycol sowie Zitronensäureester.

Darüber hinaus kann die erste Filmschicht und/oder die zweite Filmschicht als Schaum ausgeführt sein, d.h. ein eingebrachtes Gas, wie Luft, Stickstoff oder CO₂, oder ein anderes Gas enthalten.

Wie vorstehend angedeutet kann die erste Filmschicht und die zweite Filmschicht in der erfindungsgemäßen Verabreichungsform ein- oder mehrschichtig ausgeführt sein, wobei es auch möglich ist, dass die erste Filmschicht und/oder die zweite Filmschicht aus mehreren Schichten derselben Zusammensetzung aufgebaut ist, zum Beispiel indem die erste Filmschicht oder die zweite Filmschicht durch lagenweises Applizieren der Zusammensetzung übereinander hergestellt wird. Die Schichten können sich andererseits in ihrer Zusammensetzung unterscheiden, beispielsweise indem ein Pigment oder ein UV-absorbierendes Mittel in einer Schicht der ersten Filmschicht oder zweiten Filmschicht eingebracht wird und von einer Zusammensetzung ohne Pigmente oder UV-absorbierendem Mittel über- bzw. unterschichtet wird.

Bei einem mehrschichtigen Aufbau können eine oder mehrere der Schichten als Schaum ausgeführt sein, d.h. ein eingebrachtes Gas, wie Luft, Stickstoff oder CO₂, oder ein anderes Gas enthalten.

Darüber hinaus kann die erfindungsgemäße Verabreichungsform auch so ausgestaltet sein, dass diese zwei räumlich voneinander getrennte Hohlräume aufweist. So kann zum Beispiel die erste Filmschicht über eine zusätzliche Siegelung im Bereich ihrer Fläche mit der zweiten Filmschicht verbunden sein. Dadurch lassen sich zwei oder mehrere Hohlräume zwischen den Filmschichten ausbilden, die gleiche oder unterschiedliche Füllungen aufweisen können.

Dies ist insbesondere dann von Vorteil, wenn als pharmazeutischer Wirkstoff eine Base eingesetzt wird, die als solche nicht lagerstabil, aber mucosagängig ist, während das Salz des pharmazeutischen Wirkstoffs besser lagerstabil, aber nicht mucosagängig ist. In diesem Fall ist es möglich, das Salz des pharmazeutischen Wirkstoffs in einen ersten Hohlraum der erfindungsgemäßen Verabreichungsform einzubringen und eine Hilfsbase in einen zweiten Hohlraum der erfindungsgemäße Verabreichungsform einzubringen, wobei bei Applikation der Verabreichungsform in die Mundhöhle die Hilfsbase und das Salz des pharmazeutischen Wirkstoffs freigesetzt wird, so dass sich die nicht lagerstabile, aber mucosagängige Base des pharmazeutischen Wirkstoffs bilden kann. Eine Verbindung der ersten und der zweiten Filmschicht im Bereich ihrer Fläche kann auch durch eine Peelnaht vorgenommen werden, die durch Kneten der Verabreichungsform vor der Applikation aufgetrennt wird und so eine Mischung der Füllung des ersten Hohlraums mit der Füllung des zweiten Hohlraums erlaubt.

Zur Vergrößerung des zwischen den beiden Filmschichten der erfindungsgemäßen Verabreichungsform vorliegenden Volumens ist es zudem möglich, dass die erste Filmschicht und/oder die zweite Filmschicht eine nicht planare Form aufweist. Hierzu kann die erste Filmschicht oder die zweite Filmschicht bevorzugt tiefgezogen werden, um mehr Füllvolumen bei gleicher Grundfläche zu erhalten.

Des Weiteren ist es möglich, dass die erfindungsgemäße Verabreichungsform neben dem pharmazeutischen Wirkstoff, der in den Hohlraum eingebracht wird, auch in die Filmschichten eingebrachten pharmazeutischen Wirkstoff enthält. Schließlich ist es möglich, dass der in den Hohlraum eingebrachte pharmazeutische Wirkstoff in verschiedenen Modifikationen eingebracht wird, zum Beispiel ein Teil in einer direkt freisetzenden Form, während ein anderer Teil in granulierter Form oder in retardiert freisetzender Form eingebracht wird, um eine Mischkinetik der Freisetzung des pharmazeutischen Wirkstoffs zu realisieren.

Die erfindungsgemäße Verabreichungsform eignet sind insbesondere für eine orale Verabreichung, einschließlich einer buccalen, ginigvalen oder sublingualen Verabreichung, von pharmazeutischen Wirkstoffen oder einer Verabreichung am Gaumen.

Ein weiterer Aspekt der vorliegenden Erfindung betrifft schließlich ein Verfahren zur Herstellung einer Verabreichungsform der vorstehend geschilderten Art, wobei das Verfahren folgende Schritte umfasst:
a) Positionieren einer ersten und einer zweiten Filmschicht übereinander,
b) Befestigen der ersten Filmschicht auf der zweiten Filmschicht in einer Weise, dass sich mindestens eine Tasche zwischen der ersten Filmschicht und der zweiten Filmschicht ausbildet,
c) gegebenenfalls Schneiden der in b) erhaltenen Filmdoppelschichten unter Erhalt einzelner Taschen,
d) Füllen der mindestens eine Tasche mit einem pharmazeutischen Wirkstoff, und
e) Schließen der Tasche(n).

Das Befestigen im Schritt b) und/oder das Schließen der Tasche (im Schritt e)) erfolgt im Rahmen dieses Verfahrens bevorzugt durch Verkleben oder Versiegeln.

Das Positionieren einer ersten Filmschicht und einer zweiten Filmschicht übereinander in Schritt a) kann erfolgen, indem entweder zwei individuelle Filme übereinander positioniert werden oder indem ein Film in seiner Mitte geknickt wird, so dass sich zwei übereinander liegende Filmschichten ausbilden, die an einer Kante miteinander verbunden sind.

Im Folgenden soll die vorliegende Erfindung anhand einiger Beispiele näher illustriert werden, die jedoch nicht als maßgeblich für den Schutzumfang der Anmeldung zu interpretieren sind.

### Beispiel 1

Polymerfilme mit den in Tabelle 1 angegebenen Zusammensetzungen wurden formuliert und mit einer Füllung, wie in Tabelle 1 angegeben, versehen. Hierzu wurden die jeweiligen Polymerfilme zunächst aus Lösungen der genannten Inhaltsstoffe mit Hilfe eines Streichkastens beschichtet, die zu einem Film getrocknet wurden. Anschließend wurden entsprechende Filmstücke ausgestanzt, und durch Knicken wurde eine Doppelschicht mit den in Tabelle 1 angegebenen Dimensionen erzeugt. Die so erzeugte Doppelschicht wurde zur Erzeugung einer Tasche an zwei der Kanten des Films durch Heißsiegelung miteinander verbunden. Anschließend wurde die Füllung eingefüllt und die erhaltene Tasche wurde an der offenen Kante ebenfalls durch Heißsiegelung verschlossen.

**Tabelle 1**

| | | **A** | **B** | **C** |
|---|---|---|---|---|
| **Polymerfilm 1** | | | | |
| | Polyvinylalkohol | 96,9 % | 96,9 % | 96,9 % |
| | Farbstoff | 0,1 % | 0,1 % | 0,1 % |
| | Geschmacksstoff | 1 % | 1 % | 1 % |
| | Süßungsmittel | 2 % | 2 % | 2 % |
| | Flächengewicht | 48 g/m² | 48 g/m² | 48 g/m² |

| **Polymerfilm** 2 | | wie Polymerfilm 1 | wie Polymerfilm 1 | wie Polymerfilm 1 |
|---|---|---|---|---|
| **Füllung** | | | | |
| | Amberlite IPR-64 | 96 % | | |
| | Geschmacksstoff | 1 % | | |
| | Süßungsmittel | 3 % | | |
| | Miglyol | | 100% | 33% |
| | Lactose | | | 66% |
| | Menge Füllung | 100 mg | 70 µl | 150 mg |
| **Größe Pocket** | | 20 x 25 mm | 20 x 25 mm | 20 x 25 mm |

In Beispiel C wurde Miglyol als flüssiger Modelwirkstoff mit Lactose als Bindemittel formuliert. Dies erleichtert das Versiegeln zur vollständig geschlossenen Tasche, da das Bindemittel das Eindringen von flüssigem Material in den Siegelungsbereich verhindert. Somit kann durch das Bindemittel eine Schwächung der Siegelung durch das flüssige Material vermieden werden.

### Beispiel 2

Die folgenden Dextromethorphan-haltigen Verabreichungsformen wurden analog der in Beispiel 1 geschilderten Vorgehensweise hergestellt. Die Zusammensetzungen dieser Verabreichungsformen sind in der folgenden Tabelle 2 wiedergegeben.

**Tabelle 2**

| | | **A** | **B** | **C** |
|---|---|---|---|---|
| **Polymerfilm 1** | | | | |
| | Polyvinylalkohol | 96,9 % | | |
| | Polyol N10 | | 95,9 % | |
| | Pullulan | | | 95,6 % |
| | Xanthan | | | 0,3 % |
| | Farbstoff | 0,1 % | 0,1 % | 0,1 % |
| | Geschmacksstoff | 1 % | 1 % | 1 % |
| | Süßungsmittel | 2 % | 3 % | 3 % |
| | Flächengewicht | 45 g/m² | 45 g/m² | 45 g/m² |

| **Polymerfilm 2** | | wie Polymerfilm 1 | wie Polymerfilm 1 | wie Polymerfilm 1 |
|---|---|---|---|---|
| **Füllung** | | | | |
| | Dextromethorphan/ Amberlite 64 1:1 | 96 % | 96 % | 96 % |
| | Geschmacksstoff | 1 % | 1 % | 1 % |
| | Süßungsmittel | 3 % | 3 % | 3 % |
| | Menge Füllung | 52 mg | 52 mg | 52 mq |
| **Größe Pocket** | | 20 x 25 mm | 20 x 25 mm | 20 x 25 mm |

## Patentansprüche

1. Verabreichungsform für einen Wirkstoff zur Auflösung in der Mundhöhle, umfassend eine erste Filmschicht und eine über der ersten Filmschicht angeordnete zweite Filmschicht, wobei die Zusammensetzung der ersten Filmschicht mit der der zweiten Filmschicht identisch sein kann und ein wasserlösliches Polymer umfasst, wobei die erste und die zweite Filmschicht über ihre überlappenden Kanten unter Bildung mindestens eines Hohlraums miteinander verbunden sind und wobei der Hohlraum mit einem pharmazeutischen Wirkstoff gefüllt ist, wobei Nikotin als pharmazeutischer Wirkstoff ausgeschlossen ist.

2. Verabreichungsform nach Anspruch 1, **dadurch gekennzeichnet, dass** die erste und die zweite Filmschicht über ihre überlappenden Kanten miteinander verklebt oder versiegelt sind.

3. Verabreichungsform nach Anspruch 1 oder 2, **dadurch gekennzeichnet, dass** die Zusammensetzungen der ersten und der zweiten Filmschicht identisch sind.

4. Verabreichungsform nach Anspruch 1 oder 2, **dadurch gekennzeichnet, dass** die erste Filmschicht als mucoadhäsive Filmschicht ausgebildet ist und sich die zweite Filmschicht in der Mundhöhle langsamer auflöst als die erste Filmschicht.

5. Verabreichungsform nach mindestens einem der Ansprüche 1 bis 4, **dadurch gekennzeichnet, dass** der Wirkstoff in flüssiger, pulverförmiger, Granulat-, mikro- oder nanopartikulärer, oder mikro- oder nanoverkapselter Form im Hohlraum der Verabreichungsform vorliegt.

6. Verabreichungsform nach mindestens einem der vorstehenden Ansprüche, **dadurch gekennzeichnet, dass** die Verabreichungsform eine wirkstoff-haltige Füllung in einer Menge von 50 bis 1000 mg enthält.

7. Verabreichungsform nach mindestens einem der vorstehenden Ansprüche, **dadurch gekennzeichnet, dass** das wasserlösliche Polymer in der ersten und zweiten Filmschicht ausgewählt ist aus der Gruppe umfassend Polyvinylalkohol, Polyethylenglykol, Polyethylenoxid, Cellulosen, Pullulan, Gelatine und Agar.

8. Verabreichungsform nach mindestens einem der vorstehenden Ansprüche, **dadurch gekennzeichnet, dass** diese mindestens einen Hilfsstoff ausgewählt aus der Gruppe, umfassend Farbstoffe, Aromastoffe, Süßstoffe, geschmacksmaskierende Mittel, Tenside, Enhancer, pH-Regulatoren, Konservierungsmittel und/oder Antioxidationsmittel, als Bestandteil einer oder beider Filmschichten oder des Hohlraums umfasst.

9. Verabreichungsform nach Anspruch 8, **dadurch gekennzeichnet, dass** diese ein geschmacksmaskierendes Mittel als Bestandteil der ersten und/oder der zweiten Filmschicht enthält.

10. Verabreichungsform nach mindestens einem der vorstehenden Ansprüche, **dadurch gekennzeichnet, dass** die erste und/oder die zweite Filmschicht mindestens ein Pigment und/oder ein UV-absorbierendes Mittel enthält.

11. Verabreichungsform nach mindestens einem der vorstehenden Ansprüche, **dadurch gekennzeichnet, dass** die erste und/oder die zweite Filmschicht einoder mehrschichtig ausgeführt sind.

12. Verabreichungsform nach mindestens einem der vorstehenden Ansprüche, **dadurch gekennzeichnet, dass** die erste und/oder die zweite Filmschicht mindestens einen Weichmacher und/oder ein Feuchthaltemittel enthalten, bevorzugt ausgewählt aus der Gruppe, umfassend Glycerin, Propylenglycol, Polyethylenglycol und Citronensäureester.

13. Verabreichungsform nach mindestens einem der vorstehenden Ansprüche, **dadurch gekennzeichnet, dass** die erste und/oder die zweite Filmschicht über eine zusätzliche Siegelung im Bereich ihrer Fläche miteinander verbunden sind, so dass sich mindestens zwei Hohlräume zwischen den Filmschichten ausbilden, und wobei die Hohlräume gleiche oder unterschiedliche Füllungen aufweisen.

14. Verabreichungsform nach mindestens einem der vorstehenden Ansprüche, **dadurch gekennzeichnet, dass** die erste und/oder die zweite Filmschicht eine nichtplanare Form aufweist und bevorzugt als tiefgezogener Film vorliegt.

15. Verfahren zur Herstellung einer Verabreichungsform gemäß einem der Ansprüche 1 bis 14 umfassend:
a) Positionieren einer ersten Filmschicht und einer zweiten Filmschicht übereinander,
b) Befestigen der ersten Filmschicht auf der zweiten Filmschicht in einer Weise, dass sich mindestens eine Tasche zwischen der ersten Filmschicht und der zweiten Filmschicht ausbildet,
c) gegebenenfalls Schneiden der in b) erhaltenen Filmdoppelschichten unter Erhalt einzelner Taschen,
d) Füllen der mindestens eine Tasche mit einem Wirkstoff, und
e) Schließen der Tasche(n).

16. Verfahren nach Anspruch 15, wobei das Befestigen in Schritt b) und/oder das Schließen der Tasche(n) in Schritt e) durch Verkleben oder Versiegeln erfolgt.

## Claims

1. A dosage form for an active ingredient to be dissolved in the oral cavity, comprising a first film layer and a second film layer that is arranged over the first film layer, wherein the composition of the first film layer can be identical to that of the second film layer and comprises a water-soluble polymer, the first and second film layers being interconnected by their overlapping edges to form at least one cavity, and the cavity being filled with an active pharmaceutical ingredient, wherein nicotine is excluded as active pharmaceutical ingredient.

2. The dosage form according to claim 1, **characterised in that** the first and second film layers are glued or sealed together by their overlapping edges.

3. The dosage form according to claim 1 or claim 2, **characterised in that** the compositions of the first and second film layers are identical.

4. The dosage form according to claim 1 or claim 2, **characterised in that** the first film layer is formed as a mucoadhesive film layer and the second film layer dissolves more slowly in the oral cavity than the first film layer.

5. The dosage form according to at least one of claims 1 to 4, **characterised in that** the active ingredient is present in liquid, powdery, granular, micro- or nanoparticulate, or micro- or nano-encapsulated form in the cavity of the dosage form.

6. The dosage form according to at least one of the preceding claims, **characterised in that** the dosage form contains an active ingredient-containing filling in a quantity of 50 to 1000 mg.

7. The dosage form according to at least one of the preceding claims, **characterised in that** the water-soluble polymer in the first and second film layer is selected from the group comprising polyvinyl alcohol, polyethylene glycol, polyethylene oxide, celluloses, pullulan, gelatin and agar.

8. The dosage form according to at least one of the preceding claims, **characterised in that** it comprises at least one auxiliary selected from the group comprising dyes, flavourings, sweeteners, taste-masking agents, surfactants, enhancers, pH regulators, preservatives and/or antioxidants, as a component of one or both film layers or of the cavity.

9. The dosage form according to claim 8, **characterised in that** it contains a taste-masking agent as part of the first and/or second film layer.

10. The dosage form according to at least one of the preceding claims, **characterised in that** the first and/or the second film layer contains at least one pigment and/or a UV-absorbing agent.

11. The dosage form according to at least one of the preceding claims, **characterised in that** the first and/or the second film layer is/are formed with one or more layers.

12. The dosage form according to at least one of the preceding claims, **characterised in that** the first and/or the second film layer contain at least one plasticiser and/or a humectant, preferably selected from the group comprising glycerol, propylene glycol, polyethylene glycol and citric acid ester.

13. The dosage form according to at least one of the preceding claims, **characterised in that** the first and/or the second film layer are interconnected in the region of their surface by means of an additional seal, so that at least two cavities are formed between the film layers, and wherein the cavities have identical or different fillings.

14. The dosage form according to at least one of the preceding claims, **characterised in that** the first and/or the second film layer has a non-planar form and is preferably in the form of a deep-drawn film.

15. A method for producing a dosage form according to any one of claims 1 to 14, the method comprising the following steps:
a) positioning a first and a second film layer one over the other,
b) fastening the first film layer to the second film layer in such a way that at least one pouch is formed between the first film layer and the second film layer,
c) if necessary, cutting the film double layers obtained in b) to obtain individual pouches,
d) filling the at least one pouch with an active ingredient, and
e) closing the pouch(es).

16. A method according to claim 15, wherein the fastening in step b) and/or the closing of the pouch(es) in step e) are/is achieved by gluing or sealing.

## Revendications

1. Forme galénique pour un principe actif destiné à se dissoudre dans la cavité buccale, comprenant une première couche de film et une deuxième couche de film disposée par-dessus la première couche de film, la composition de la première couche de film pouvant être identique à celle de la deuxième couche de film et comprenant un polymère hydrosoluble, les première et deuxième couches de film étant reliées l'une à l'autre au niveau de leurs bords qui se chevauchent en formant au moins une cavité, et ladite cavité étant remplie d'un principe actif pharmaceutique, la nicotine étant exclue en tant que principe actif pharmaceutique.

2. Forme d'administration selon la revendication 1, **caractérisée en ce que** la première et la deuxième couches de film sont collées ou scellées l'une à l'autre au niveau de leurs bords qui se chevauchent.

3. Forme d'administration selon la revendication 1 ou 2, **caractérisée en ce que** les compositions de la première et de la deuxième couches de film sont identiques.

4. Forme d'administration selon la revendication 1 ou 2, **caractérisée en ce que** la première couche de film est conçue comme une couche de film mucoadhésive et **en ce que** la deuxième couche de film se dissout plus lentement dans la cavité buccale que la première couche de film.

5. Forme d'administration selon au moins l'une des revendications 1 à 4, **caractérisée en ce que** le principe actif se présente sous forme liquide, pulvérulente, granulée, micro- ou nanoparticulaire, ou micro- ou nanoencapsulée dans la cavité de la forme d'administration.

6. Forme d'administration selon au moins l'une des revendications précédentes, **caractérisée en ce que** la forme d'administration contient une charge contenant le principe actif en une quantité comprise entre 50 et 1000 mg.

7. Forme d'administration selon au moins l'une des revendications précédentes, **caractérisée en ce que** le polymère hydrosoluble présent dans les première et deuxième couches de film est choisi dans le groupe comprenant l'alcool polyvinylique, le polyéthylène glycol, le poly(oxyde d'éthylène), les celluloses, le pullulane, la gélatine et l'agar.

8. Forme d'administration selon au moins l'une des revendications précédentes, **caractérisée en ce qu'**elle comprend au moins un excipient choisi dans le groupe comprenant les colorants, les arômes, les édulcorants, les agents masquant le goût, les tensioactifs, les exhausteurs, les régulateurs de pH, les conservateurs et/ou les antioxydants, en tant que composant de l'une ou des deux couches de film ou de la cavité.

9. Forme d'administration selon la revendication 8, **caractérisée en ce qu'**elle contient un agent masquant le goût en tant que composant de la première et/ou de la deuxième couche de film.

10. Forme d'administration selon au moins l'une des revendications précédentes, **caractérisée en ce que** la première et/ou la deuxième couche de film contient au moins un pigment et/ou un agent absorbant les UV.

11. Forme d'administration selon au moins l'une des revendications précédentes, **caractérisée en ce que** la première et/ou la deuxième couche de film sont réalisées en une ou plusieurs couches.

12. Forme d'administration selon au moins l'une des revendications précédentes, **caractérisée en ce que** la première et/ou la deuxième couche de film contiennent au moins un plastifiant et/ou un agent humidifiant, de préférence choisis dans le groupe comprenant la glycérine, le propylène glycol, le polyéthylène glycol et les esters de l'acide citrique.

13. Forme d'administration selon au moins l'une des revendications précédentes, **caractérisée en ce que** la première et/ou la deuxième couche de film sont reliées entre elles par un scellage supplémentaire au niveau de leur surface, de sorte qu'au moins deux cavités se forment entre les couches de film, et dans laquelle les cavités présentent des remplissages identiques ou différents.

14. Forme d'administration selon au moins l'une des revendications précédentes, **caractérisée en ce que** la première et/ou la deuxième couche de film présente une forme non plane et se présente de préférence sous la forme d'un film embouti.

15. Procédé de fabrication d'une forme d'administration selon l'une des revendications 1 à 14, comprenant :
a) le positionnement d'une première couche de film et d'une deuxième couche de film l'une sur l'autre,
b) la fixation de la première couche de film sur la deuxième couche de film de manière à former au moins une poche entre la première couche de film et la deuxième couche de film,
c) le cas échéant, la découpe des doubles couches de film obtenues en b) pour obtenir des poches individuelles,
d) le remplissage de ladite au moins une poche avec un principe actif, et
e) la fermeture de la ou des poches.

16. Procédé selon la revendication 15, dans lequel la fixation à l'étape b) et/ou la fermeture de la ou des poches à l'étape e) s'effectuent par collage ou scellage.
